(19) Europäisches Patentamt

European Patent Office

Office européen des brevets

(11) **EP 0 579 776 B1**

(12) **EUROPEAN PATENT SPECIFICATION**

(45) Date of publication and mention
of the grant of the patent:
**20.01.1999 Bulletin 1999/03**

(21) Application number: **92914476.4**

(22) Date of filing: **10.06.1992**

(51) Int. Cl.$^6$: **B01J 21/00**, C01B 35/10,
C07C 2/10, C07C 4/06,
C07C 6/00

(86) International application number:
**PCT/US92/04864**

(87) International publication number:
**WO 93/00165 (07.01.1993 Gazette 1993/02)**

(54) **OLEFIN OLIGOMERIZATION PROCESS**

VERFAHREN ZUR OLIGOMERISIERUNG VON OLEFINEN

PROCEDE D'OLIGOMERISATION D'OLEFINES

(84) Designated Contracting States:
**BE DE FR GB IT NL**

(30) Priority: **21.06.1991 US 718879**
**21.06.1991 US 718884**
**21.06.1991 US 718893**

(43) Date of publication of application:
**26.01.1994 Bulletin 1994/04**

(73) Proprietor:
**MOBIL OIL CORPORATION**
**Fairfax, Virginia 22037-0001 (US)**

(72) Inventors:
• **BHORE, Nazeer, Ahmed**
**Londonberry, DE 19810 (US)**
• **LE, Quang, Ngoc**
**Cherry Hill, NJ 08002 (US)**
• **THOMSON, Robert, Thomas**
**Lawrenceville, NJ 08648 (US)**
• **YOKOMIZO, Grant, Hugh**
**Cherry Hill, NJ 08003 (US)**

(74) Representative: **Kador & Partner**
**Corneliusstrasse 15**
**80469 München (DE)**

(56) References cited:
**US-A- 4 673 559**    **US-A- 4 849 186**
**US-A- 4 880 611**    **US-A- 4 956 514**
**US-A- 4 982 046**    **US-A- 5 057 296**
**US-A- 5 100 533**

• **Jour. Catalysis, Vol. 80, 1983 (USA), J.P. VAN DEN BERG et al., "Low Temperature Oligomerization of Small Olefins on Zeolite H-ZSM-5. An Investigation with High Resolution Solid State T3C-NMR", pp 130-138 (see entire document).**
• **Nature, Vol. 306, 1983 (England), P.B. MOORE, "An X-ray structural study of cacoxanite, a mineral phosphate", pp 396-398 (see entire document).**
• **Jour. Catalysis, Vol. 80, 1983 (USA), J.P. VAN DEN BERG et al., "Reaction of Small Olefins on Zeolite H-ZSM-5. A Thermogravimetric Study at Low and Intermediate Temperatures", pp 130-144 (see entire document).**

**Description**

This invention relates to an olefin oligomerization process.

Conversion of olefins to gasoline and/or distillate products is disclosed in U.S. Patents 3,960,978 and 4,021,502, wherein gaseous olefins in the range of ethylene to pentene, either alone or in admixture with paraffins are converted into an olefinic gasoline blending stock by contacting the olefins with a ZSM-5 catalyst.

In the process for catalytic conversion of olefins to heavier hydrocarbons by catalytic oligomerization using a medium pore shape selective acid crystalline zeolite, such as ZSM-5, process conditions can be varied to favor the formation of hydrocarbons of varying molecular weight. At moderate temperature and relatively high pressure, the conversion conditions favor $C_{10}^+$ aliphatic product. Lower olefinic feedstocks containing $C_2$-$C_8$ alkenes may be converted; however, the distillate mode conditions of the prior art do not convert a major fraction of ethylene. A typical reactive feedstock consists essentially of $C_3$-$C_6$ mono-olefins, with varying amounts of nonreactive paraffins and the like being acceptable components for ordinary commercial purposes. These prior art oligomerization methods require relatively high temperature to provide adequate conversion, resulting in undesirable cracking reactions and a broad spectrum of carbon numbers in the products. The product of medium pore olefin catalysis can be characterized as nearly linear, with only moderate branching due to the constrained pores of the catalysts.

While low temperature oligomerization is known, prior catalysts have not shown sufficient activity below about 200°C to be practical in industrial applications. The advantages of low severity oligomerization with medium pore zeolites have been described in US Patents 4,746,762 4,873,385 and 4,956,514. It is generally understood that low temperature oligomerization can be selective to produce incremental oligomers which have molecular weights as multiples of the monomers, such as isomeric propene oligomers consisting essentially of C6, C9, C12, etc. These reactions are selective without significant cracking of the desired product; however, the relative inactivity of prior art catalysts has prevented development of low temperture processes.

It is an object of this invention to provide an improved process for selective catalytic oligomerization of an olefinic feedstock employing a novel acid solid catalyst which has high oligomerization activity at low temperature and which produces highly branched, incremental oligomers. The resulting oligomers can be further converted under disproportionation or cracking conditions to yield lower olefins rich in tertiary compounds.

Accordingly, the invention resides in a process for oligomerizing an alkene-containing feedstock by contacting the feedstock with a catalyst comprising an inorganic, porous, non-layered crystalline phase material exhibiting, after calcination, an X-ray diffraction pattern with at least one peak at a d-spacing greater than about 18 Angstrom Units characterized in having after calcination a benzene adsorption capacity of greater than 15 grams of benzene per 100 grams of said material at 6.7 kPa (50 torr) and 25°C, and having a hexagonal arrangement of uniformly-sized pores having diameters of at least about 13 Angstrom Units and, after calcination, exhibiting a hexagonal electron diffraction pattern that can be indexed with a $d_{100}$ value greater than about 18 Angstrom Units.

The catalyst employed in the process of the invention comprises an inorganic, porous, non-layered crystalline phase material exhibiting, in its calcined form, an X-ray diffraction pattern with at least one peak at a position greater than about 18 Angstrom Units d-spacing (4.909 degrees two-theta for Cu K-alpha radiation). More particularly, the calcined crystalline non-layered material of the invention may be characterized by an X-ray diffraction pattern with at least two peaks at positions greater than about 10 Angstrom Units d-spacing (8.842 degrees two-theta for Cu K-alpha radiation), at least one of which is at a position greater than about 18 Angstrom Units d-spacing, and no peaks at positions less than about 10 Angstrom units d-spacing with relative intensity greater than about 20% of the strongest peak. Still more particularly, the X-ray diffraction pattern of the calcined material of this invention will have no peaks at positions less than about 10 Angstrom units d-spacing with relative intensity greater than about 10% of the strongest peak.

X-ray diffraction data were collected on a Scintag PAD X automated diffraction system employing theta-theta geometry, Cu K-alpha radiation, and an energy dispersive X-ray detector. Use of the energy dispersive X-ray detector eliminated the need for incident or diffracted beam monochromators. Both the incident and diffracted X-ray beams were collimated by double slit incident and diffracted collimation systems. The slit sizes used, starting from the X-ray tube source, were 0.5, 1.0, 0.3 and 0.2 mm, respectively. Different slit systems may produce differing intensities for the peaks. The materials of the present invention that have the largest pore sizes may require more highly collimated incident X-ray beams in order to resolve the low angle peak from the transmitted incident X-ray beam.

The diffraction data were recorded by step-scanning at 0.04 degrees of two-theta, where theta is the Bragg angle, and a counting time of 10 seconds for each step. The interplanar spacings, d's, were calculated in Angstrom units (A), and the relative intensities of the lines, I/Io, where Io is one-hundredth of the intensity of the strongest line, above background, were derived with the use of a profile fitting routine. The intensities were uncorrected for Lorentz and polarization effects. The relative intensities are given in terms of the symbols vs = very strong (75-100), s = strong (50-74), m = medium (25-49) and w = weak (0-24). It should be understood that diffraction data listed as single lines may consist of multiple overlapping lines which under certain conditions, such as very high experimental resolution or crystallographic changes, may appear as resolved or partially resolved lines. Typically, crystallographic changes can include minor changes in unit cell parameters and/or a change in crystal symmetry, without a substantial change in structure. These minor effects, including changes in relative intensities, can also occur as a result of differences in cation content, framework composition, nature and degree of pore filling, thermal and/or hydrothermal history, and peak width/shape variations due to particle size/shape effects, structural disorder or other factors known to those skilled in the art of X-ray diffraction.

The material employed in the process of the invention is further characterised by an equilibrium benzene adsorption capacity of greater than about 15 grams benzene/100 grams crystal at 6.7 kPa (50 torr) and 25°C. The equilibrium benzene adsorption capacity characteristic of this material is measured on the basis of no pore blockage by incidental contaminants. For instance, the sorption test will be conducted on the crystalline material phase having any pore blockage contaminants and water removed by ordinary methods. Water may be removed by dehydration techniques, e.g. thermal treatment. Pore blocking inorganic amorphous materials. e.g. silica, and organics may be removed by contact with acid or base or other chemical agents such that the detrital material will be removed without detrimental effect on the crystal of the invention.

The equilibrium benzene adsorption capacity is determined by contacting the material of the invention, after dehydration or calcination at, for example, 450 to 700°C, typically 540°C, for at least one hour and other treatment, if necessary, in an attempt to remove any pore blocking contaminants, at 25°C and 6.7 kPa (50 torr) benzene until equilibrium is reached. The weight of benzene sorbed is then determined as more particularly described hereinafter.

The materials used in the process of the invention are generally mesoporous, by which is meant they have uniform pores within the size range of 13 to 200 Angstroms, more usually 15 to 100 Angstroms. In a preferred embodiment, the material appears to have a hexagonal arrangement of large channels with open internal diameters from 13 to 200 Angstroms. This structure can be revealed by transmission electron microscopy and electron diffraction. Thus, electron micrographs of properly oriented specimens of the material show a hexagonal arrangement of large channels and the corresponding electron diffraction patterns give an approximately hexagonal arrangement of diffraction maxima. The d100 spacing of the electron diffraction patterns is the distance between adjacent spots on the hkO projection of the hexagonal lattice and is related to the repeat distance a0 between channels observed in the electron micrographs through the formula d100 = a0 3/2. This $d_{100}$ spacing observed in the electron diffraction pattern corresponds to the d-spacing of the low angle peak (>18 Angstrom d-spacing) in the X-ray diffraction pattern. The most highly ordered preparations of the material obtained so far have 20-40 distinct spots observable in the electron diffraction patterns. These patterns can be indexed with the hexagonal hkO subset of unique reflections of 100, 110, 200, 210, etc., and their symmetry-related reflections.

The inorganic, non-layered mesoporous crystalline material used in this invention typically has the following composition:

$$M^n/q(Wa\ Xb\ Yc\ Zd\ Oh)$$

wherein W is a divalent element, such as a divalent first row transition metal, e.g. manganese, cobalt and iron, and/or magnesium, preferably cobalt; X is a trivalent element, such as aluminum, boron, iron and/or gallium, preferably aluminum; Y is a tetravalent element such as silicon and/or germanium, preferably silicon; Z is a pentavalent element, such as phosphorus; M is one or more ions, such as, for example, ammonium, Group IA, IIA and VIIB ions, usually hydrogen, sodium and/or fluoride ions; n is the charge of the composition excluding M expressed as oxides; q is the weighted molar average valence of M; n/q is the number of moles or mole fraction of M; a, b, c and d are mole fractions of W, X, Y and Z, respectively; h is a number of from 1 to 2.5; and (a+b+c+d) = 1 .

A preferred embodiment of the above crystalline material is when (a+b+c) is greater than d, and h = 2. A further embodiment is when a and d = 0, and h = 2.

In the as-synthesized form, the material employed in this invention typically has a composition, on an anhydrous basis, expressed empirically as follows:

$$rRMn/q(Wa\ Xb\ Yc\ Zd\ Oh)$$

wherein R is the total organic material not included in M as an ion, and r is the coefficient for R, i.e. the number of moles or mole fraction of R. The M and R components are associated with the material as a result of their presence during crystallization, and are easily removed or, in the case of M, replaced by conventional post-crystallization methods.

Preferably, the crystalline material is a metallosilicate which is synthesized with Bronsted acid active sites by incorporating a tetrahedrally coordinated trivalent element, such as Al, Ga, B, or Fe, within the silicate framework. Aluminosilicate materials of this type are thermally and chemically stable, properties favored for acid catalysis; however, the advantages of mesoporous structures may be utilized by employing highly siliceous materials or crystalline metallosilicate having one or more tetrahedral species having varying degrees of acidity. In addition to the preferred aluminosilicates, the gallosilicate, ferrosilicate and borosilicate materials may be employed. Although matrices may be formed with the germanium analog of silicon, these are expensive and generally no better than the metallosilicates.

Most preferably, the crystalline material is an aluminosilicate having a silica-to-alumina ratio of 5:1 to 1000:1 and significant Bronsted acid activity. Acid activity may be measured by acid cracking activity or ammonia absorption properties, such as temperature programmed desorption.

Synthesis of the crystalline material employed in the process of the invention is described in detail in our International Patent Publication No. WO 91/11390. In particular, materials having the composition defined by the above formula can be prepared from a reaction mixture having a composition in terms of mole ratios of oxides, within the following ranges:

| Reactants | Useful | | | Preferred | | |
|---|---|---|---|---|---|---|
| $X_2O_3/YO_2$ | 0 | to | 0.5 | 0.001 | to | 0.5 |
| $X_2O_3/(YO_2+Z_2O_5)$ | 0.1 | to | 100 | 0.1 | to | 20 |
| $X_2O_3/(YO_2+WO+Z_2O_5)$ | 0.1 | to | 100 | 0.1 | to | 20 |
| Solvent/ | | | | | | |
| $(YO_2+WO+Z_2O_5+X_2O_3)$ | 1 | to | 1500 | 5 | to | 1000 |
| $OH^-/YO_2$ | 0 | to | 10 | 0 | to | 5 |
| $(M_{2/e}O+R_{2/f}O)/$ | | | | | | |
| $(YO_2+WO+Z_2O_5+X_2O_3)$ | 0.01 | to | 20 | 0.05 | to | 5 |
| $M_{2/e}O/$ | | | | | | |
| $(YO_2+WO+Z_2O_5+X_2O_3)$ | 0 | to | 10 | 0 | to | 5 |
| $R_{2/f}O/$ | | | | | | |
| $(YO_2+WO+Z_2O_5+X_2O_3)$ | 0.01 | to | 2.0 | 0.03 | to | 1.0 |

wherein e and f are the weighted average valences of M and R, respectively, wherein the solvent is a $C_1$ to $C_6$ alcohol or diol, or, more preferably, water and wherein R comprises an organic directing agent having the formula $R_1R_2R_3R_4Q^+$ wherein Q is nitrogen or phosphorus and wherein at least one of $R_1$, $R_2$, $R_3$ and $R_4$ is aryl or alkyl group having 6 to 36 carbon atoms, e.g. $-C_6H_{13}$, $-C_{10}H_{21}$, $-C_{16}H_{33}$ and $-C_{18}H_{37}$, and each of the remainder of $R_1$, $R_2$, $R_3$ and $R_4$ is selected from hydrogen and an alkyl group having 1 to 5 carbon atoms. The compound from which the above ammonium or phosphonium ion is derived may be, for example, the hydroxide, halide, silicate or mixtures thereof.

The particular effectiveness of the above directing agent, when compared with other such agents known to direct synthesis of one or more other crystal structures, is believed due to its ability to function as a template in the nucleation and growth of the desired ultra-large pore materials. Non-limiting examples of these directing agents include cetyltrimethylammonium, cetyltrimethylphosphonium, octadecyltrimethylphosphonium, benzyltrimethylammonium, cetylpyridinium, myristyltrimethylammonium, decyltrimethylammonium, dodecyltrimethylammonium and dimethyldidodecylammonium compounds.

Preferably, the total organic, R, present in the reaction mixture comprises an additional organic directing agent in the form of an ammonium or phosphonium ion of the above directing agent formula but wherein each $R_1$, $R_2$, $R_3$ and $R_4$ is selected from hydrogen and an alkyl group of 1 to 5 carbon atoms (2 of the alkyl groups can be interconnected to form a cyclic compound). Examples of the additional organic directing agent include tetramethylammonium, tetraethylammonium, tetrapropylammonium, tetrabutylammonium and pyrrolidinium compounds. The molar ratio of the first-mentioned organic directing agent to the additional organic directing agent can be in the range 100/1 to 0.01/1. Where the additional organic directing agent is present, the molar ratio $R_{2/f}O/(YO_2+WO+Z_2O_5+X_2O_3)$ in the reaction mixture is preferably 0.1 to 2.0, most preferably 0.12 to 1.0.

In addition, to vary the pore size of the final crystalline phase material, the total organic, R, in the reaction mixture can include an auxiliary organic in addition to the organic directing agent(s) described above. This auxiliary organic is selected from (1) aromatic hydrocarbons and amines having 5-20 carbon atoms and halogen- and $C_1$-$C_{14}$ alkyl-substituted derivatives thereof, (2) cyclic and polycyclic aliphatic hydrocarbons and amines of 5 to 20 carbon atoms and halogen- and $C_1$-$C_{14}$ alkyl-substituted derivatives thereof and (3) straight and branched chain aliphatic hydrocarbons and amines having 3-16 carbon atoms and halogen-substituted derivatives thereof.

In the above auxiliary organics, the halogen substituent is preferably bromine. The $C_1$-$C_{14}$ alkyl substituent may be a linear or branched aliphatic chain, such as, for example, methyl, ethyl, propyl, isopropyl, butyl, pentyl and combinations thereof. Examples of these auxiliary organics include, for example, p-xylene, trimethylbenzene, triethylbenzene and triisopropylbenzene.

With the inclusion of the auxiliary organic in the reaction mixture, the mole ratio of auxiliary organic/$YO_2$ will be from 0.05 to 20, preferably from 0.1 to 10, and the mole ratio of auxiliary organic/organic directing agent(s) will be from 0.02 to 100, preferably from 0.05 to 35.

When a source of silicon is used in the synthesis method, it is preferred to use at least in part an organic silicate, such as, for example, a quaternary ammonium silicate. Non-limiting examples of such a silicate include tetramethylammonium silicate and tetraethylorthosilicate.

Non-limiting examples of various combinations of W, X, Y and Z contemplated for the above reaction mixture include:

| W | X | Y | Z |
|---|---|---|---|
| -- | Al | Si | -- |
| -- | Al | -- | P |
| -- | Al | Si | P |
| Co | Al | -- | P |
| Co | Al | Si | P |
| -- | -- | Si | -- |

including the combinations of W being Mg, or an element selected from the divalent first row transition metals, e.g. Mn, Co and Fe; X being B, Ga or Fe; and Y being Ge.

To produce the crystalline material of the invention, the reaction mixture described above is maintained at a temperature of 25 to 250°C, preferably 50 to 175°C, and preferably a pH of 9 to 14 for a period of time until the required crystals form, typically 5 minutes to 14 days, more preferably 1 to 300 hours.

When the crystalline material of the invention is an aluminosilicate, the synthesis method conveniently involves the following steps:

(1) Mix the organic (R) directing agent with the solvent or solvent mixture such that the mole ratio of solvent/$R_{2/f}O$ is within the range of 50 to 800, preferably from 50 to 500. This mixture constitutes the "primary template" for the synthesis method.
(2) To the primary template mixture of step (1) add the silica and alumina such that the ratio of $R_{2/f}O/(SiO_2+Al_2O_3)$ is within the range 0.01 to 2.0.
(3) Agitate the mixture resulting from step (2) at a temperature of 20 to 40°C, preferably for 5 minutes to 3 hours.
(4) Allow the mixture to stand with or without agitation, preferably at 20 to 50°C, and preferably for 10 minutes to 24 hours.
(5) Crystallize the product from step (4) at a temperature of 50 to 150°C, preferably for 1 to 72 hours.

The process of the invention is particularly useful for upgrading $C_2$-$C_6$ lower olefins to heavier hydrocarbons, such as highly branched C6-C20+ olefins. It is useful in oligomerizing alpha-olefins to make $C_{30}$+ lubricants, for example by reacting 1-decene to make its trimer, etc. Numerous mono-olefins, including propene, n-butenes, isobutene, pentenes, mixtures thereof, etc., can be reacted selectively in aliphatic hydrocarbon feedstocks. An advantage of the present process is reaction selectivity, such that non-olefinic products can be avoided as reaction by-products, due to the substantial absence of dehydrogenation, cyclization and alkane formation. However, the feedstocks may contain non-deleterious amounts of paraffins, naphthenes, aromatics.

Reaction temperature for oligomerization may vary widely. Below 40°C the reaction may be too slow and above 250°C selectivity may be lost for some products. The preferred range is about 40 to 250°C, especially 80 to 200°C. Pressure can also vary greatly from sub-atmospheric to very high pressures (eg 10 to 20,000 kPa), with many process reactions taking place in the 100 to 13,000 kPa range. By contrast with medium pore zeolites, it has been found that increasing pressure for a given feedstock does not result in higher molecular weight products. This is unexpected behavior for one skilled in the art of zeolitic catalysis. For instance, in propylene oligomerization the distribution of higher incremental oligomers falls dramatically above about 5000 kPa (700 psig) with increased selectivity to hexene and nonene isomers. The reaction may be conducted in the gas phase, liquid phase or dense phase.

Industrial application of the present process will ordinarily require at least 50% conversion of feedstock, preferably 80-100%, and such conversion can be obtained with continuous reactor operation using fixed bed, fluidized bed, moving bed, slurry reactor, etc. Typical space velocities, based on active catalyst are in the range of 0.1-5/hr WHSV, preferably 0.5-2.

This invention also provides a secondary process step for converting the oligomerized product, e.g., $C_6$-$C_{18}$ monoalkene oligomers to lower isoalkenes, especially $C_4$-$C_5$ tertiary alkenes, using a medium pore zeolite catalyst, such as ZSM-5 or MCM-22 (US Patent No. 4954325). The second stage olefin cracking or disproportionation process step is conveniently effected at a temperature of 250 to 700°C, preferably 300 to 450°C, a pressure of 100 to 1500 kPa, preferably 150 to 500 kPa and an WHSV of 0.1 to 100 $hr^{-1}$, preferably 0.2 to 20 $hr^{-1}$. Interstage separation may be used to recover unreacted lower aliphatics as byproduct or recycle, or the second reaction stage may employ unfractionated primary stage effluent as a cascade feedstream for disproportionation/cracking reactions in contact with the medium pore zeolite catalyst.

In the examples metric units and parts by weight are employed unless otherwise indicated.

## EXAMPLE 1

One hundred grams of cetyltrimethylammonium (CTMA) hydroxide solution, prepared by contacting a 29 wt.% N,N,N- trimethyl-1-hexadecanaminium chloride solution with a hydroxide-for-halide exchange resin, was combined with 100 grams of an aqueous solution of tetramethylammonium (TMA) silicate (10% silica) with stirring. Twenty-five grams of HiSil, a precipitated hydrated silica containing about 6 wt.% free water and about 4.5 wt.% bound water of hydration and having an ultimate particle size of about 0.02 micron, was added. The resulting mixture was placed in a polypropylene bottle, which was kept in a steam box at 95°C overnight. The mixture had a composition in terms of moles per mole $Al_2O_3$:

$$
\begin{array}{lll}
2.7 & \text{moles} & Na_2O \\
392 & \text{moles} & SiO_2 \\
35.7 & \text{moles} & (CTMA)_2O \\
61.7 & \text{moles} & (TMA)_2O \\
6231 & \text{moles} & H_2O
\end{array}
$$

The resulting solid product was recovered by filtration and dried in air at ambient temperature. The product was then calcined at 540°C for 1 hour in nitrogen, followed by 6 hours in air.

The calcined product proved to have a surface area of 475 $m^2$/g and the following equilibrium adsorption capacities in grams/100 grams:

$$
\begin{array}{ll}
H_2O & 8.3 \\
\text{Cyclohexane} & 22.9 \\
\text{n-Hexane} & 18.2 \\
\text{Benzene} & 21.5
\end{array}
$$

The product of this example may be characterized as including a very strong relative intensity line at 37.8 ± 2.0 Angstroms d-spacing, and weak lines at 21.6 ± 1.0 and 19.2 ± 1.0 Angstroms. The present ultra-large pore material was demonstrated to be in the product of this example by transmission electron microscopy (TEM), which produced images of a hexagonal arrangement of uniform pores and hexagonal electron diffraction pattern with a $d_{100}$ value of about 39 Angstroms.

## EXAMPLE 2

One hundred grams of cetyltrimethylammonium (CTMA) hydroxide solution prepared as in Example 1 was combined with 100 grams of an aqueous solution of tetramethylammonium (TMA) hydroxide (25%) with stirring. Twenty-five grams of HiSil, a precipitated hydrated silica containing about 6 wt.% free water and about 4.5 wt.% bound water of hydration and having an ultimate particle size of about 0.02 micron, was added. The resulting mixture was placed in a static autoclave at 150°C overnight. The mixture had a composition in terms of moles per mole $Al_2O_3$:

$$
\begin{array}{rll}
2.7 & \text{moles} & Na_2O \\
291 & \text{moles} & SiO_2 \\
35.7 & \text{moles} & (CTMA)_2O \\
102 & \text{moles} & (TMA)_2O \\
6120 & \text{moles} & H_2O
\end{array}
$$

The resulting solid product was recovered by filtration and dried in air at ambient temperature. The product was then calcined at 540°C for 1 hour in nitrogen, followed by 6 hours in air.

The calcined product proved to have a surface area of 993 $m^2/g$ and the following equilibrium adsorption capacities in grams/100 grams:

$$
\begin{array}{ll}
H_2O & 7.1 \\
\text{Cyclohexane} & 47.2 \\
\text{n-Hexane} & 36.2 \\
\text{Benzene} & 49.5
\end{array}
$$

The X-ray diffraction pattern of the calcined product of this example is characterized as including a very strong relative intensity line at $39.3 \pm 2.0$ Angstroms d-spacing, and weak lines at $22.2 \pm 1.0$ and $19.4 \pm 1.0$ Angstroms. TEM indicated that the product contained the present ultra-large pore material.

A portion of the above product was then contacted with 100% steam at 788°C (1450°F) for two hours. The surface area of the steamed material was measured to be 440 $m^2/g$, indicating that about 45% was retained following severe steaming.

Another portion of the calcined product of this example was contacted with 100% steam at 677°C (1250°F) for two hours. The surface area of this material was measured to be 718 $m^2/g$, indicating that 72% was retained after steaming at these conditions.

## EXAMPLE 3

Water, cetyltrimethylammonium hydroxide solution prepared as in Example 1, aluminum sulfate, HiSil and an aqueous solution of tetrapropylammonium (TPA) bromide (35%) were combined to produce a mixture having a composition in terms of moles per mole $Al_2O_3$:

$$
\begin{array}{rll}
0.65 & \text{moles} & Na_2O \\
65 & \text{moles} & SiO_2 \\
8.8 & \text{moles} & (CTMA)_2O \\
1.22 & \text{moles} & (TPA)_2O \\
1336 & \text{moles} & H_2O
\end{array}
$$

The resulting mixture was placed in a polypropylene bottle, which was kept in a steam box at 95°C for 192 hours. The sample was then cooled to room temperature and combined with CTMA hydroxide solution prepared as in Example 1 and TMA hydroxide (25% by weight) in the weight ratio of 3 parts mixture, 1 part CTMA hydroxide and 2 parts TMA hydroxide. The combined mixture was then placed in a polypropylene bottle and kept in a steam box at 95°C overnight. The combined mixture had a composition in terms of moles per mole $Al_2O_3$:

$$
\begin{array}{rll}
0.65 & \text{moles} & Na_2O \\
65 & \text{moles} & SiO_2 \\
15 & \text{moles} & (CTMA)_2O \\
1.22 & \text{moles} & (TPA)_2O \\
35.6 & \text{moles} & (TMA)_2O \\
2927 & \text{moles} & H_2O
\end{array}
$$

The resulting solid product was recovered by filtration and dried in air at ambient temperature. The product was then calcined at 540°C for 1 hour in nitrogen, followed by 6 hours in air.

The calcined product proved to have a surface area of 1085 $m^2$/g and the following equilibrium adsorption capacities in grams/100 grams:

$$
\begin{array}{ll}
H_2O & 11.5 \\
\text{Cyclohexane} & > 50 \\
\text{n-Hexane} & 39.8 \\
\text{Benzene} & 62
\end{array}
$$

The X-ray diffraction pattern of the calcined product of this example is characterized as including a very strong relative intensity line at $38.2 \pm 2.0$ Angstroms d-spacing, and weak lines at $22.2 \pm 1.0$ and $19.4 \pm 1.0$ Angstroms. TEM indicated the product contained the present ultra-large pore material.

### EXAMPLE 4

Two hundred grams of cetyltrimethylammonium (CTMA) hydroxide solution prepared as in Example 1 was combined with 2 grams of Catapal alumina (alpha-alumina monohydrate, 74% alumina) and 100 grams of an aqueous solution of tetramethylammonium (TMA) silicate (10% silica) with stirring. Twenty-five grams of HiSil, a precipitated hydrated silica containing about 6 wt.% free water and about 4.5 wt.% bound water of hydration and having an ultimate particle size of about 0.02 micron, was added. The resulting mixture was placed in a static autoclave at 150°C for 48 hours. The mixture had a composition in terms of moles per mole $Al_2O_3$:

$$
\begin{array}{rll}
0.23 & \text{moles} & Na_2O \\
33.2 & \text{moles} & SiO_2 \\
6.1 & \text{moles} & (CTMA)_2O \\
5.2 & \text{moles} & (TMA)_2O \\
780 & \text{moles} & H_2O
\end{array}
$$

The resulting solid product was recovered by filtration and dried in air at ambient temperature. The product was then calcined at 540°C for 1 hour in nitrogen, followed by 6 hours in air.

The calcined product proved to have a surface area of 1043 $m^2$/g and the following equilibrium adsorption capacities in grams/100 grams:

| | |
|---|---|
| $H_2O$ | 6.3 |
| Cyclohexane | > 50 |
| n-Hexane | 49.1 |
| Benzene | 66.7 |

The X-ray diffraction pattern of the calcined product of this example is characterized as including a very strong relative intensity line at $40.8 \pm 2.0$ Angstroms d-spacing, and weak lines at $23.1 \pm 1.0$ and $20.1 \pm 1.0$ Angstroms. TEM indicated that the product contained the present ultra-large pore material (see Example 23).

**EXAMPLE 5**

Two-hundred sixty grams of water was combined with 77 grams of phosphoric acid (85%), 46 grams of Catapal alumina (74% alumina), and 24 grams of pyrrolidine (Pyr) with stirring. This first mixture was placed in a stirred autoclave and heated to 150°C for six days. The material was filtered, washed and air-dried. Fifty grams of this product was slurried with 200 grams of water and 200 grams of cetyltrimethylammonium hydroxide solution prepared as in Example 1. Four hundred grams of an aqueous solution of tetraethylammonium silicate (10% silica) was then added to form a second mixture which was placed in a polypropylene bottle and kept in a steam box at 95°C overnight. The first mixture had a composition in terms of moles per mole $Al_2O_3$:

| | | |
|---|---|---|
| 1.0 | moles | $P_2O_5$ |
| 0.51 | moles | $(Pyr)_2O$ |
| 47.2 | moles | $H_2O$ |

The resulting solid product was recovered by filtration and dried in air at ambient temperature. The product was then calcined at 540°C for 1 hour in nitrogen, followed by 6 hours in air.

The calcined product proved to have a surface area of 707 $m^2$/g and the following equilibrium adsorption capacities in grams/100 grams:

| | |
|---|---|
| $H_2O$ | 33.2 |
| Cyclohexane | 19.7 |
| n-Hexane | 20.1 |
| Benzene | 23.3 |

The X-ray diffraction pattern of the calcined product of this example is characterized as including a very strong relative intensity line at $25.4 \pm 1.5$ Angstroms d-spacing. TEM indicated the product contained the present ultra-large pore material (see Example 23).

**EXAMPLE 6**

A solution of 1.35 grams of $NaAlO_2$ (43.5% $Al_2O_3$, 30% $Na_2O$) dissolved in 45.2 grams of water was mixed with 17.3 grams of NaOH, 125.3 grams of colloidal silica (40%, Ludox HS-40) and 42.6 grams of 40% aqueous solution of tetraethylammonium (TEA) hydroxide. After stirring overnight, the mixture was heated for 7 days in a steam box (95°C). Following filtration, 151 grams of this solution was mixed with 31 grams of cetyltrimethylammonium hydroxide solution prepared as in Example 1 and stored in the steam box at 95°C for 13 days. The mixture had the following relative molar composition:

$$0.25 \quad \text{moles} \quad Al_2O_3$$
$$10 \quad \text{moles} \quad Na_2O$$
$$36 \quad \text{moles} \quad SiO_2$$
$$0.95 \quad \text{moles} \quad (CTMA)_2O$$
$$2.5 \quad \text{moles} \quad (TEA)_2O$$
$$445 \quad \text{moles} \quad H_2O$$

The resulting solid product was recovered by filtration and washed with water and ethanol. The product was then calcined at 540°C for 1 hour in nitrogen, followed by 6 hours in air.

The calcined product composition included 0.14 wt.% Na, 68.5 wt.% $SiO_2$ and 5.1 wt.% $Al_2O_3$, and proved to have a benzene equilibrium adsorption capacity of 58.6 grams/100 grams.

The X-ray diffraction pattern of the calcined product of this example is characterized as including a very strong relative intensity line at 31.4 ± 1.5 Angstroms d-spacing. TEM indicated that the product contained the present ultra-large pore material.

### EXAMPLE 7

A mixture of 300 grams of cetyltrimethylammonium (CTMA) hydroxide solution prepared as in Example 1 and 41 grams of colloidal silica (40%, Ludox HS-40) was heated in a 600 cc autoclave at 150°C for 48 hours with stirring at 200 rpm. The mixture has a composition in terms of moles per mole $SiO_2$:

The resulting solid product was recovered by filtration, washed with water, then calcined at 540°C for 1 hour in nitrogen, followed by 10 hours in air.

The calcined product composition included less than 0.01 wt.% Na, about 98.7 wt.% $SiO_2$ and about 0.01 wt.% $Al_2O_3$, and proved to have a surface area of 896 $m^2/g$. The calcined product had the following equilibrium adsorption capacities in grams/100 grams:

$$H_2O \quad\quad\quad 8.4$$
$$\text{Cyclohexane} \quad\quad\quad 49.8$$
$$\text{n-Hexane} \quad\quad\quad 42.3$$
$$\text{Benzene} \quad\quad\quad 55.7$$

The X-ray diffraction pattern of the calcined product of this example is characterized as including a very strong relative intensity line at 40.0 ± 2.0 Angstroms d-spacing and a weak line at 21.2 ± 1.0 Angstroms. TEM indicated that the product of this example contained at least three separate phases, one of which was the present ultra-large pore material.

### EXAMPLE 8

A mixture of 150 grams of cetyltrimethylammonium (CTMA) hydroxide solution prepared as in Example 1 and 21 grams of colloidal silica (40%, Ludox HS-40) with an initial pH of 12.64 was heated in a 300 cc autoclave at 150°C for 48 hours with stirring at 200 rpm. The mixture had a composition in terms of moles per mole $SiO_2$:

$$0.5 \quad \text{mole} \quad (CTMA)_2O$$
$$46.5 \quad \text{moles} \quad H_2O$$

The resulting solid product was recovered by filtration, washed with water, then calcined at 540°C for 6 hours in air.

The calcined product composition was measured to include 0.01 wt.% Na, 93.2 wt.% $SiO_2$ and 0.016 wt.% $Al_2O_3$, and proved to have a surface area of 992 $m^2/g$ and the following equilibrium adsorption capacities in grams/100 grams:

| | |
|---|---|
| $H_2O$ | 4.6 |
| Cyclohexane | > 50 |
| n-Hexane | > 50 |
| Benzene | 62.7 |

The X-ray diffraction pattern of the calcined product of this example is characterized as including a very strong relative intensity line at 43.6 ± 2.0 Angstroms d-spacing and weak lines at 25.1 ± 1.5 and 21.7 ± 1.0 Angstroms. TEM indicated that the product contained the present ultra-large pore material.

## EXAMPLE 9

Sodium aluminate (4.15g) was added slowly into a solution containing 16g of myristyltrimethylammonium bromide ($C_{14}$TMABr) in 100g of water. Tetramethylammonium silicate (100g-10% $SiO_2$), HiSil (25g) and tetramethylammonium hydroxide (14.2g-25% solution) were then added to the mixture. The mixture was crystallized in an autoclave at 120°C with stirring for 24 hours.

The product was filtered, washed and air dried. Elemental analysis showed the product contained 53.3 wt% $SiO_2$, 3.2 wt% $Al_2O_3$, 15.0 wt% C, 1.88 wt% N, 0.11 wt% Na and 53.5 wt% ash at 1000°C. The X-ray diffraction pattern of the material (calcined at 540°C for 1 hour in $N_2$ and 6 hours in air) includes a very strong relative intensity line at 35.3 ± 2.0 Angstroms d-spacing and weak lines at 20.4 ± 1.0 and 17.7 ± 1.0 Angstroms d-spacing. TEM indicated that the product contained the present ultra-large pore material.

The washed product, having been exchanged with 1N ammonium nitrate solution at room temperature, then calcined, proved to have a surface area of 827 $m^2/g$ and the following equilibrium adsorption capacities in g/100g anhydrous sorbent:

| | |
|---|---|
| $H_2O$ | 30.8 |
| Cyclohexane | 33.0 |
| n-Hexane | 27.9 |
| Benzene | 40.7 |

## EXAMPLE 10

Sodium aluminum (4.15g) was added slowly into a solution containing 480g of dodecyltrimethylammonium hydroxide ($C_{12}$TMAOH, 50%) solution diluted with 120g of water. UltraSil (50g) and an aqueous solution of tetramethylammonium silicate (200g-10% $SiO_2$) and tetramethylammonium hydroxide (26.38g-25% solution) were then added to the mixture. The mixture was crystallized in an autoclave at 100°C with stirring for 24 hours. The product was filtered, washed and air dried. The X-ray diffraction pattern of the material (calcined at 540°C for 1 hour in $N_2$ and 6 hours in air) includes a very strong relative intensity line at 30.4 ± 1.5 Angstroms d-spacing and weak lines at 17.7 ± 1.0 and 15.3 ± 1.0 Angstroms d-spacing. TEM indicated that the product contained the present ultra-large pore material.

The washed product, having been exchanged with 1N ammonium nitrate solution at room temperature, then calcined, proved to have a surface area of 1078 $m^2/g$ and the following equilibrium adsorption capacities in g/100g anhydrous sorbent:

|  |  |
|---|---|
| $H_2O$ | 32.6 |
| Cyclohexane | 38.1 |
| n-Hexane | 33.3 |
| Benzene | 42.9 |

## EXAMPLE 11

A solution of 4.9 grams of $NaAlO_2$ (43.5 % $Al_2O_3$, 30% $NaO_2$) in 37.5 grams of water was mixed with 46.3 cc of 40% aqueous tetraethylammonium hydroxide solution and 96 grams of colloidal silica (40%, Ludox HS-40). The gel was stirred vigorously for 0.5 hour, mixed with an equal volume (150 ml) of cetyltrimethylammonium hydroxide solution prepared as in Example 1 and reacted at 100°C for 168 hours. The mixture had the following composition in terms of moles per mole $Al_2O_3$:

|  |  |  |
|---|---|---|
| 1.1 | moles | $Na_2O$ |
| 30.6 | moles | $SiO_2$ |
| 3.0 | moles | $(TEA)_2O$ |
| 3.25 | moles | $(CTMA)_2O$ |
| 609 | moles | $H_2O$ |

The resulting solid product was recovered by filtration, washed with water then calcined at 540°C for 16 hours in air. The calcined product proved to have a surface area of 1352 $m^2$/g and the following equilibrium adsorption capacities in grams/100 grams:

|  |  |
|---|---|
| $H_2O$ | 23.6 |
| Cyclohexane | >50 |
| n-Hexane | 49 |
| Benzene | 67.5 |

The X-ray diffraction pattern of the calcined product of this example may be characterized as including a very strong relative intensity line at 38.5 ± 2.0 Angstroms d-spacing and a weak line at 20.3 ± 1.0 Angstroms. TEM indicated that the product contained the present ultra-large pore material.

## EXAMPLE 12

Two hundred grams of cetyltrimethylammonium (CTMA) hydroxide solution prepared as in Example 1 was combined with 4.15 grams of sodium aluminate and 100 grams of aqueous tetramethylammonium (TMA) silicate solution (10% silica) with stirring. Twenty-five grams of HiSil, a precipitated hydrated silica containing about 6 wt.% free water and about 4.5 wt.% bound water of hydration and having an ultimate particle size of about 0.02 micron, was added. The resulting mixture was placed in a static autoclave at 150°C for 24 hours. The mixture had a composition in terms of moles per mole $Al_2O_3$:

$$1.25 \quad \text{moles} \quad Na_2O$$
$$27.8 \quad \text{moles} \quad SiO_2$$
$$5.1 \quad \text{moles} \quad (CTMA)_2O$$
$$4.40 \quad \text{moles} \quad (TMA)_2O$$
$$650 \quad \text{moles} \quad H_2O$$

The resulting solid product was recovered by filtration and dried in air at ambient temperature. The product was then calcined at 540°C for 1 hour in nitrogen, followed by 6 hours in air. TEM indicated that this product contained the present ultra-large pore material. The X-ray diffraction pattern of the calcined product of this example can be characterized as including a very strong relative intensity line at $44.2 \pm 2.0$ Angstroms d-spacing and weak lines at $25.2 \pm 1.5$ and $22.0 \pm 1.0$ Angstroms.

The calcined product proved to have a surface area of 932 $m^2$/g and the following equilibrium adsorption capacities in grams/100 grams:

| | |
|---|---|
| $H_2O$ | 39.3 |
| Cyclohexane | 46.6 |
| n-Hexane | 37.5 |
| Benzene | 50 |

The product of this example was then ammonium exchanged with 1 N $NH_4NO_3$ solution, followed by calcination at 540°C for 10 hours in air.

## EXAMPLE 13

Two hundred grams of cetyltrimethylammonium (CTMA) hydroxide solution prepared as in Example 1 was combined with 4.15 grams of sodium aluminate and 100 grams of aqueous tetramethylammonium (TMA) silicate solution (10% silica) with stirring. Twenty-five grams of HiSil, a precipitated hydrated silica containing about 6 wt.% free water and about 4.5 wt.% bound water of hydration and having an ultimate particle size of about 0.02 micron, was added. The resulting mixture was placed in a steam box at 100°C for 48 hours. The mixture had a composition in terms of moles per mole $Al_2O_3$:

$$1.25 \quad \text{moles} \quad Na_2O$$
$$27.8 \quad \text{moles} \quad SiO_2$$
$$5.1 \quad \text{moles} \quad (CTMA)_2O$$
$$4.4 \quad \text{moles} \quad (TMA)_2O$$
$$650 \quad \text{moles} \quad H_2O$$

The resulting solid product was recovered by filtration and dried in air at ambient temperature. The product was then calcined at 540°C for 1 hour in nitrogen, followed by 6 hours in air.

The calcined product proved to have the following equilibrium adsorption capacities in grams/100 grams:

```
H₂O                  35.2
Cyclohexane       >  50
n-Hexane             40.8
Benzene              53.5
```

The X-ray diffraction pattern of the calcined product of this example may be characterized as including a very strong relative intensity line at $39.1 \pm 2.0$ Angstroms d-spacing and weak lines at $22.4 \pm 1.0$ and $19.4 \pm 1.0$ Angstroms. TEM indicated that this product contained the present ultra-large pore material.

The product of this example was then ammonium exchanged with 1 N $NH_4NO_3$ solution, followed by calcination at 540°C for 10 hours in air.

## EXAMPLE 14

A mixture of 125 grams of 29% CTMA chloride aqueous solution, 200 grams of water, 3 grams of sodium aluminate (in 50 grams $H_2O$), 65 grams of Ultrasil, amorphous precipitated silica available from PQ Corporation, and 21 grams NaOH (in 50 grams $H_2O$) was stirred thoroughly and crystallized at 150°C for 168 hours. The reaction mixture had the following relative molar composition in terms of moles per mole silica:

```
 0.10   moles   (CTMA)₂O
21.89   moles   H₂O
 0.036  moles   NaAlO₂
 0.53   moles   NaOH
```

The solid product was isolated by filtration, washed with water, dried for 16 hours at room temperature and calcined at 540°C for 10 hours in air.

The calcined product proved to have a surface area of 840 $m^2$/g, and the following equilibrium adsorption capacities in grams/100 grams:

```
H₂O                  15.2
Cyclohexane          42.0
n-Hexane             26.5
Benzene              62
```

The X-ray diffraction pattern of the calcined product of this Example may be characterized as including a very strong relative intensity line at $40.5 \pm 2.0$ Angstroms d-spacing. TEM indicated that the product contained the present ultra-large pore material.

## EXAMPLE 15

To make the primary template mixture for this example, 240 grams of water was added to a 92 gram solution of 50% dodecyltrimethylammonium hydroxide, 36% isopropyl alcohol and 14% water such that the mole ratio of Solvent/$R_{2/f}O$ was 155. The mole ratio of $H_2O/R_{2/f}O$ in this mixture was 149 and the IPA/$R_{2/f}O$ mole ratio was 6. To the primary template mixture was added 4.15 grams of sodium aluminate, 25 grams of HiSil, 100 grams of aqueous tetramethylammonium silicate solution (10% $SiO_2$) and 13.2 grams of 25% aqueous tetramethylammonium hydroxide solution. The mole ratio of $R_{2/f}O/(SiO_2+Al_2O_3)$ was 0.28 for the mixture.

This mixture was stirred at 25°C for 1 hour. The resulting mixture was then placed in an autoclave at 100°C and stirred at 100 rpm for 24 hours. The mixture in the autoclave had the following relative molar composition in terms of moles per mole $SiO_2$:

$$
\begin{array}{lll}
0.05 & \text{mole} & Na_2O \\
0.036 & \text{mole} & Al_2O_3 \\
0.18 & \text{mole} & (C_{12}TMA)_2O \\
0.12 & \text{mole} & (TMA)_2O \\
36.0 & \text{moles} & H_2O \\
1.0 & \text{mole} & IPA
\end{array}
$$

The resulting solid product was recovered by filtration, washed with water and dried in air at ambient temperature. The product was then calcined at 540°C for 1 hour in nitrogen, followed by 6 hours in air.

The calcined product proved to have a surface area of 1223 $m^2$/g and the following equilibrium adsorption capacities in grams/100 grams:

$$
\begin{array}{ll}
H_2O & 25.5 \\
\text{Cyclohexane} & 41.1 \\
\text{n-Hexane} & 35.1 \\
\text{Benzene} & 51
\end{array}
$$

The X-ray diffraction pattern of the calcined product of this example is characterized as including a very strong relative intensity line at 30.8 ± 1.5 Angstroms d-spacing and weak lines at 17.9 ± 1.0 and 15.5 ± 1.0 Angstroms. TEM indicated this product to contain the present ultra-large pore material.

## EXAMPLE 16

A 50.75 gram quantity of decyltrimethylammonium hydroxide (prepared by contacting a ca. 29 wt.% solution of decyltrimethylammonium bromide with a hydroxide-for-halide exchange resin) was combined with 8.75 grams of tetraethylorthosilicate. The mixture was stirred for about 1 hour and then transferred to a polypropylene jar which was then placed in a steambox for about 24 hours. The mixture had a composition in terms of moles per mole $SiO_2$:

$$
\begin{array}{lll}
0.81 & \text{mole} & (C_{10}TMA)_2O \\
47.6 & \text{moles} & H_2O
\end{array}
$$

The resulting solid product was filtered and washed several times with warm (60-70°C) distilled water and with acetone. The final product was calcined to 538°C in $N_2$/air mixture and then held in air for about 8 hours.

The calcined product proved to have a surface area of 915 $m^2$/g and an equilibrium benzene adsorption capacity of 35 grams/100 grams. Argon physisorption data indicated an argon uptake of 0.34 cc/gram, and a pore size of 15 Angstroms.

The X-ray diffraction pattern of the calcined product of this example may be characterized as including a very strong relative intensity line at 27.5 ± 1.5 Angstroms d-spacing and weak lines at 15.8 ± 1.0 and 13.7 ± 1.0 Angstroms. TEM indicated that the product of this example contained the present ultra-large pore material.

## EXAMPLE 17

To eighty grams of cetyltrimethylammonium hydroxide (CTMAOH) solution prepared as in Example 1 was added 1.65 grams of $NaAlO_2$. The mixture was stirred at room temperature until the $NaAlO_2$ was dissolved. To this solution was added 40 grams of aqueous tetramethylammonium (TMA) silicate solution (10 wt.% $SiO_2$), 10 grams of HiSil, 200 grams of water and 70 grams of 1,3,5-trimethylbenzene (mesitylene). The resulting mixture was stirred at room temperature for several minutes. The gel was then loaded into a 600 cc autoclave and heated at 105°C for sixty-eight hours with stirring at 150 rpm. The mixture had a composition in terms of moles per mole $Al_2O_3$:

$$1.25 \text{ moles } Na_2O$$
$$27.8 \text{ moles } SiO_2$$
$$5.1 \text{ moles } (CTMA)_2O$$
$$2.24 \text{ moles } (TMA)_2O$$
$$2256 \text{ moles } H_2O$$

The resulting product was filtered and washed several times with warm (60-70°C) distilled water and with acetone. The final product was calcined to 538°C in $N_2$/air mixture and then held in air for about 10 hours.

The calcined product proved to have an equilbrium benzene adsorption capacity of >25 grams/100 grams.

The X-ray diffraction pattern of the calcined product of this example may be characterized as including a broad, very strong relative intensity line at about 102 Angstroms d-spacing, but accurate positions of lines in the extreme low angle region of the X-ray diffraction pattern are very difficult to determine with conventional X-ray diffractometers. Furthermore, finer collimating slits were required to resolve a peak at this low 2-theta angle. The slits used in this example, starting at the X-ray tube, were 0.1, 0.3, 0.5 and 0.2 mm, respectively. TEM indicated that the product of this example contained several materials with different $d_{100}$ values as observed in their electron diffraction patterns. These materials were found to possess $d_{100}$ values between about 85 Angstroms d-spacing and about 120 Angstroms d-spacing.

### EXAMPLE 18

To eighty grams of cetyltrimethylammonium hydroxide (CTMAOH) solution prepared as in Example 1 was added 1.65 grams of $NaAlO_2$. The mixture was stirred at room temperature until the $NaAlO_2$ was dissolved. To this solution was added 40 grams of aqueous tetramethylammonium (TMA) silicate solution (10 wt.% $SiO_2$), 10 grams of HiSil, 200 grams of water and 120 grams of 1,3,5-trimethylbenzene (mesitylene). The resulting mixture was stirred at room temperature for several minutes. The gel was then loaded into a 600 cc autoclave and heated at 105°C for ninety hours with stirring at 150 rpm. The mixture had a composition in terms of moles per mole $Al_2O_3$:

$$1.25 \text{ moles } Na_2O$$
$$27.8 \text{ moles } SiO_2$$
$$5.1 \text{ moles } (CTMA)_2O$$
$$2.24 \text{ moles } (TMA)_2O$$
$$2256 \text{ moles } H_2O$$
$$132.7 \text{ moles } 1,3,5\text{-trimethylbenzene}$$

The resulting product was filtered and washed several times with warm (60-70°C) distilled water and with acetone. The final product was calcined to 538°C in $N_2$/air mixture and then held in air for about 10 hours.

The calcined product proved to have a surface area of 915 $m^2$/g and an equilbrium benzene adsorption capacity of >25 grams/100 grams. Argon physisorption data indicated an argon uptake of 0.95 cc/gram, and a pore size centered on 78 Angstroms (Dollimore-Heal Method) but running from 70 to greater than 105 Angstoms.

The X-ray diffraction pattern of the calcined product of this example may be characterized as having only enhanced scattered intensity in the very low angle region of the X-ray diffraction, where intensity from the transmitted incident X-ray beam is usually observed. However, TEM indicated that the product of this example contained several materials with different $d_{100}$ values as observed in their electron diffraction patterns. These materials were found to possess $d_{100}$ values between about 85 Angstroms d-spacing and about 110 Angstroms d-spacing.

## EXAMPLE 19

To eighty grams of cetyltrimethylammonium hydroxide (CTMAOH) solution prepared as in Example 1 was added 1.65 grams of $NaAlO_2$. The mixture was stirred at room temperature until the $NaAlO_2$ was dissolved. To this solution was added 40 grams of aqueous tetramethylammonium (TMA) silicate solution (10 wt.% $SiO_2$), 10 grams of HiSil, and 18 grams of 1,3,5-trimethylbenzene (mesitylene). The resulting mixture was stirred at room temperature for several minutes. The gel was then loaded into a 300 cc autoclave and heated at 105°C for four hours with stirring at 150 rpm. The mixture had a composition in terms of moles per mole $Al_2O_3$:

$$
\begin{array}{lll}
1.25 & \text{moles} & Na_2O \\
27.8 & \text{moles} & SiO_2 \\
5.1 & \text{moles} & (CTMA)_2O \\
2.24 & \text{moles} & (TMA)_2O \\
650 & \text{moles} & H_2O \\
19.9 & \text{moles} & 1,3,5\text{-trimethylbenzene}
\end{array}
$$

The resulting product was filtered and washed several times with warm (60-70°C) distilled water and with acetone. The final product was calcined to 538°C in $N_2$/air mixture and then held in air for about 8 hours.

The calcined product proved to have a surface area of 975 $m^2$/g and an equilbrium benzene adsorption capacity of >40 grams/100 grams. Argon physisorption data indicated an argon uptake of 0.97 cc/gram, and a pore size of 63 Angstroms (Dollimore-Heal Method), with the peak occurring at $P/P_o$=0.65.

The X-ray diffraction pattern of the calcined product of this example may be characterized as including a very strong relative intensity line at $63 \pm 5$ Angstroms d-spacing and weak lines at $36.4 \pm 2.0$, $31.3 \pm 1.5$ Angstroms and $23.8 \pm 1.0$ Angstroms d-spacing. TEM indicated that the product of this example contained the present ultra-large pore material.

## EXAMPLE 20(a)

### Argon Physisorption For Pore Systems Up to About 60 Angstroms Diameter

To determine the pore diameters of the products of this invention with pores up to about 60 Angstroms in diameter, 0.2 gram samples of the products of Examples 1 through 16 were placed in glass sample tubes and attached to a physisorption apparatus as described in U.S. Patent No. 4,762,010.

The samples were heated to 300°C for 3 hours in vacuo to remove adsorbed water. Thereafter, the samples were cooled to -186°C (87°K) by immersion of the sample tubes in liquid argon. Metered amounts of gaseous argon were then admitted to the samples in stepwise manner as described in U.S. Patent No. 4,762,010, column 20. From the amount of argon admitted to the samples and the amount of argon left in the gas space above the samples, the amount of argon adsorbed can be calculated. For this calculation, the ideal gas law and the calibrated sample volumes were used. (See also S.J. Gregg et al., Adsorption, Surface Area and Porosity, 2nd ed., Academic Press, 1982). It is common to use relative pressures which are obtained by forming the ratio of the equilibrium pressure and the vapor pressure $P_o$ of the adsorbate at the temperature where the isotherm is measured. Sufficiently small amounts of argon were admitted in each step to generate 168 data points in the relative pressure range from 0 to 0.6. At least about 100 points are required to define the isotherm with sufficient detail.

The step (inflection) in the isotherm, in this case (Example 4 product) at about $P/P_o = 0.4$, indicates filling of a pore system. The size of the step indicates the amount adsorbed, whereas the position of the step in terms of $P/P_o$ reflects the size of the pores in which the adsorption takes place. Larger pores are filled at higher $P/P_o$. In order to better locate the position of the step in the isotherm, the derivative with respect to log $(P/P_o)$ is formed. There is further provided a physical scale on the axis which converts the position of an adsorption peak in terms of log $(P/P_o)$ to the physical pore diameter in Angstroms. This conversion was obtained by using the following formula:

$$\log(P/P_O) = \frac{K}{d-0.38}\left[\frac{S^4}{3(L-D/2)^3} - \frac{S^{10}}{9(L-D/2)^9} - \frac{S^4}{3(D/2)^3} + \frac{S^{10}}{9(D/2)^9}\right]$$

wherein d = pore diameter in nanometers, K = 32.17, S = 0.2446, L = d + 0.19, and D = 0.57.

This formula is derived from the method of Horvath and Kawazoe (G. Horvath et al., J. Chem. Eng. Japan, 16 (6) 470(1983)). The constants required for the implementation of this formula were determined from a measured isotherm of ALPO-5 and its known pore size. This method is particularly useful for microporous materials having pores of up to about 60 Angstroms in diameter.

The pore size of the material of Example 4 is 39.6 Angstroms with the peak occurring at log $(P/P_o)$ = -0.4 or $P/P_o$ = 0.4, while the pore size of the material from U.S. Patent 4,880,611 is 12 Angstroms or $P/P_o$ = 0.02. In the other materials, a peak is observed at $P/P_o$ = 0.015 which is denoted by an asterisk in Figure 17. This peak reflects adsorption on the walls of the pores and is not otherwise indicative of the size of the pores of a given material. A value of $P/P_o$ of 0.03 corresponds to 13 Angstroms pore size.

The results of this procedure for the samples from Examples 1 through 17 are tabulated below. The samples from Examples 10, 13 and 16 gave two separate peaks, believed to be the result of two separate ultra-large pore phases in the products.

| Examples | Pore Diameter, Angstroms |
|---|---|
| 1 | 32.2 |
| 2 | 35.4 |
| 3 | 42.5 |
| 4 | 39.6 |
| 5 | 16.9 |
| 6 | 27.3 |
| 7 | 36.6 |
| 8 | 42.6 |
| 9 | 28.3 |
| 10 | 22.8, 30.8 |
| 11 | 36.8 |
| 12 | 36.1 |
| 13 | 35.0, 42.1 |
| 14 | 40.0 |
| 15 | 22.4, 30.4 |
| 16 | 15.0 |

## PROCESS EXAMPLE A

To demonstrate the catalytic oligomerization properties of the crystalline material of the invention, hereinafter referred to as MCM-41, a catalyst prepared from the calcined product of Example 11 was compared with the known zeolite catalysts, ZSM-5 and ZSM-23, in the oligomerization of propylene. The experiments were performed in a fixed bed isothermal tubular reactor at temperatures varying from 80°C to 160°C (175°F-320°F), propene partial pressures between 1800 and 10,400 kPa (250-1500 psig) and weight hourly space velocities between 0.25 and 1.0 (based on active catalyst). The results are shown in Tables A and B.

## TABLE A

| Catalyst | kPa (psig) | Temp.(°C/F) | WHSV | % Conv. $C_3=$ |
|----------|-----------|-------------|------|----------------|
| ZSM-5 | 3551 (500) | 204/400 | 0.4 | 98 |
| ZSM-23 | 3551 (500) | 185/365 | 0.25 | 91 |
| MCM-41 | 3551 (500) | 96/205 | 1.0 | 84 |
| MCM-41 | 3551 (500) | 96/205 | 0.25 | >98 |

## TABLE B

| Catalyst | ZSM-23 | MCM-41 |
|----------|--------|--------|
| Propylene Conv.,Wt% | 91 | 98 |
| Product Comp., wt% | | |
| $C_6=$ | 59 | 2 |
| $C_9=$ | 20 | 43 |
| $C_{12}=$ | 11 | 34 |
| $C_{15}=$ | 7 | 12 |
| $C_{18}=$ | 2 | 5 |
| $C_{21}=$ | 1 | 3 |

The data contained in Table A show that the ultra large pore MCM-41 zeolite exhibits significantly improved activity compared with ZSM-5 and ZSM-23 for propylene oligomerization. MCM-41 also provides significantly higher $C_9$ and $C_{12}$ hydrocarbon yield for propylene oligomerization as shown in Table B.

The ability of ultra large pore MCM-41 zeolites to selectively oligomerize olefins, especially propylene, at low temperature (i.e. <100°C) to trimers and tetramers is unexpected and represents a significant improvement over previous catalysts. The high activity allows for control of branching index by varying the process parameters such as pressure and feed flowrate.

In the process of the invention, increasing temperature results in higher MW product formation. Increasing pressure causes an increase in activity and a shift in selectivity to lower MW products as shown in Table C.

## TABLE C

| Pressure kPa | Temp(°C/°F) | WHSV | Conv | Olefin Selectivity | | | | | |
|------|-------------|------|------|-------|-------|----------|----------|----------|----------|
| | | | | $C_6$ | $C_9$ | $C_{12}$ | $C_{15}$ | $C_{18}$ | $C_{21}$ |
| 1826 (250 psig) | 96/205 | 0.25 | 75% | 5 | 38 | 36 | 18 | 2 | 1 |
| 1826 (250 psig) | 96/205 | 1.00 | 18% | 17 | 41 | 31 | 10 | 1 | 0 |
| 7001 (1000 psig) | 96/205 | 1.00 | 85% | 8 | 60 | 25 | 6 | 1 | 0 |

As shown in Table D, the catalyst of the invention exhibits excellent short-term stability, showing no activity loss during a 29 days on stream (DOS) at 96°C, 7000 kPa (1000 psig) and 1 WHSV.

## TABLE D

### Olefin Selectivity

| DOS | Conv | $C_6$ | $C_9$ | $C_{12}$ | $C_{15}$ | $C_{18}$ |
|-----|------|-------|-------|----------|----------|----------|
| 11  | 85%  | 8     | 62    | 23       | 6        | 1        |
| 29  | 84%  | 8     | 60    | 25       | 6        | 1        |

High pressures are employed to maximize olefin conversion, while low temperatures are desirable to minimize side reactions such as oligomer cracking, cyclization, and hydrogen transfer.

## PROCESS EXAMPLE B

The procedure of Example A was repeated with acid MCM-41 catalyst for conversion of propene at 121°C (250°F), 7000 kPa (1000 psig) and 1/hr WHSV and the $C_6$ hexene fraction was analyzed to show the isomer distribution. In Table E these isomers are compared with those produced by (A) FCC gas oil cracking over standard zeolite Y and (B) naphtha (decene) cracking over zeolite MCM-22. While methylpentene isomers are the dominant product in all cases, H-MCM-41 (Ex.C-C) gives about 20% of the highly branched isomers, including an unusually high (17%) selectivity to 2,3-dimethyl-2-butene. The tertiary-olefins are valuable intermediates in organic syntheses, especially clean fuel additive manufacture.

## TABLE E

### Hexene Distribution (%)

| ISOMER | (A) | (B) | (C) |
|---|---|---|---|
| 1-Hexene | 5.66 | 1.88 | 0.00 |
| C-2-Hexene | 7.50 | 4.45 | 0.30 |
| t-2-Hexene | 13.16 | 8.16 | 0.57 |
| C-3-Hexene | 1.84 | 1.11 | 0.60 |
| t-3-Hexene | 6.99 | 4.22 | |
| 2-M-1-Pnt | 8.37 | 9.51 | 6.07 |
| 3-M-1-Pnt | 2.30 | 2.14 | |
| 4-M-1-Pnt | 1.65 | 1.55 | |
| 2-M-2-Pnt | 16.98 | 17.50 | 30.75 |
| 3-M-Cis-2-Pnt | 8.45 | 10.61 | 4.11 |
| 3-M-tr-2-Pnt | 13.65 | 17.40 | 8.56 |
| 4-M-Cis-2-Pnt | 1.53 | 1.75 | 4.51 |
| 4-M-tr-2-Pnt | ? | 5.33 | 19.83 |
| 2-Eth-1-But | 3.03 | 3.35 | 0.50 |
| 2,3-DM-1-But | 2.15 | 4.15 | 2.28 |
| 3,3-DM-1-But | 0.70 | 0.00 | 0.00 |
| 2,3-DM-2-But | 6.05 | 6.89 | 16.88 |

## PROCESS EXAMPLE C

In order to demonstrate the synthesis of poly (alpha-olefin), a lubricant range 1-decene was contacted with MCM-41 catalyst prepared according to Example 11. Table F gives the reaction conditions for an isothermal reactor at 101 kPa and 15 parts 1-decene per part of catalyst.

## TABLE F

### 1-Decene Oligomerization Over Ultra Large Pore Materials

| Catalyst | Temp. °C | Time hr. | $C_{10}$ conv. | $\%C_{10}$ | $\%C_{20}$ | $\%C_{30}$ | $\%C_{40}$ |
|---|---|---|---|---|---|---|---|
| MCM-41 | RT | | 0 | 100 | 0 | 0 | 0 |
| | 120 | 0 | 7 | 93 | 7 | 0 | 0 |
| | 120 | 1 | 13.9 | 86.1 | 13.9 | 0 | 0 |
| | 120 | 2 | 17.2 | 82.8 | 17.2 | 0 | 0 |
| | 120 | 18.3 | 22.9 | 77.1 | 22.1 | 0 | 0 |
| | 150 | 0 | 25.3 | 74.7 | 23.0 | 2.3 | 0 |
| | 150 | 1 | 27.9 | 72.1 | 25.3 | 2.6 | 0 |
| | 150 | 2 | 30.3 | 69.7 | 27.0 | 3.3 | 0 |
| | 150 | 18.6 | 63.8 | 36.2 | 52.2 | 10.0 | 1.6 |
| | 150 | 24 | 71.4 | 28.6 | 59.0 | 12.1 | 2.3 |
| | 180 | 0 | 71.8 | 28.2 | 56.4 | 12.5 | 2.9 |
| | 180 | 1 | 77.3 | 22.7 | 59.6 | 14.3 | 3.4 |
| | 180 | 1.75 | 77.2 | 22.8 | 58.2 | 15.3 | 3.7 |

The $C_{30}$-$C_{40}$ olefinic oligomer product is hydrogenated to provide a poly (alpha-olefin) synthetic lubricant. The decene monomers and dimers can be recycled back to the oligomerization reactor to increase the yield of $C_{30}^{+}$ products.

## PROCESS EXAMPLE D

A propene-containing $C_3$ refinery stream was converted selectively employing MCM-41 catalyst prepared according to Example 13 in an isothermal tubular reactor at about 120°C (250°F), 10400 kPa (1500 psig) and 1.8 WHSV, based on active catalyst. The results are given in Table G:

**TABLE G**

| | |
|---|---|
| Conversion, wt% | 87 |
| Selectivity, wt% | |
| $C_6$ Olefins | 11 |
| $C_9$ Olefins | 59 |
| $C_{12}$ Olefins | 20 |
| $C_{15}$ Olefins | 5 |
| $C_{18}^+$ Olefins | 5 |

As shown in Table G, the $C_6^+$ products consisted primarily of propylene trimers and tetramers. The reaction effluent from the primary stage oligomerization was separated by cooling, flashing and phase separation to recover a gaseous stream comprising mainly unconverted $C_3$ aliphatics and a condensed liquid stream consisting essentially of $C_6^+$ branched olefins. The liquid stream was cracked using a steamed MCM-22 catalyst in a secondary stage reactor at 425°C (900°F), 450 kPa (50 psig) total pressure, 6 WHSV oligomer feed, and sufficient nitrogen (2000 GHSV) to maintain hydrocarbon contact times near one second. The results are displayed in Table H:

**TABLE H**

| | |
|---|---|
| Reactor Effluent, wt% | |
| Total $C_1$-$C_2$ | 0.7 |
| Propylene | 16.0 |
| Propane | 0.3 |
| Isobutylene | 14.8 |
| Linear Butenes | 14.7 |
| Isoamylenes | 19.5 |
| Linear Pentenes | 8.6 |
| $C_4$-$C_5$ Paraffins | 2.2 |
| $C_6$ Olefins | 10.2 |
| $C_6$ Paraffins + Cyclics | 1.2 |
| Total $C_7^+$ | 11.6 |

As shown in Table G, the cracking of the $C_6^+$ olefins yielded 16.0 wt% propylene and only 0.3 wt% propane, thus providing the highly-olefinic $C_3$ stream. The other products derived from this reaction were primarily $C_4^+$ olefins, which may be recycled to the cracking reactor to enhance propylene yield. However, olefins in the effluent stream may also be employed for the production of clean fuels.

The secondary stage reaction effluent may be separated conventionally by distillation or the like to recover an olefinic product stream rich in propene and $C_4$-$C_5$ tertiary olefins, which may be converted to MTBE and/or TAME; and wherein a $C_6^+$ heavy hydrocarbon may be recovered to recycle for further conversion and/or interstage separation. It is feasible to recover propene and butenes as an enriched olefin product stream, with a $C_5^+$ stream rich in tertiary olefins being partially etherified as a gasoline blending component.

**PROCESS EXAMPLE E**

Using low pressure/short contact time conditions, MCM-22 catalyst was preconditioned with decene feedstock at 427°C (800°F), 450 kPa (50 psig), and 6 WHSV for 72 hours to reach steady state condition. Thereafter the $C_6$-$C_{18}$ oligomer feedstream from the primary stage effluent of Example D was reacted at 69% conversion. As seen in Table I, the product stream contained high yields of $C_3$-$C_5$ olefins and low amounts of paraffins.

<center>TABLE I</center>

| | |
|---|---|
| Conv. to $C_5^-$ (wt%) | 69 |

$C_5^-$ Product Dist., wt%

| | |
|---|---|
| Total $C_1$-$C_2$ | 0.4 |
| Propylene | 16.8 |
| Isobutylene | 22.3 |
| Linear Butenes | 17.4 |
| Isoamylenes | 28.7 |
| Linear Pentenes | 11.0 |
| $C_3$-$C_5$ Paraffins | 3.4 |

Among the $C_4$ olefins, the ratio of isobutylene to linear butenes (1.28) exceeded the equilibrium value (0.97), while branched/linear ratio for the $C_5$ olefins (2.61) was lower than that expected at equilibrium (3.18). Within the $C_6^=$ fraction eluting from the cracking reactor, high yields of branched hexenes were found, with only minor amounts of $C_6$ cyclic compounds formed during the two-stage conversion of propylene.

Table J compares the combined results of the two-stage propylene conversion process of Examples D and E with those of a conventional one-stage propylene conversion process over ZSM-5 (conducted at 350°C, 170 kPa and 10 WHSV).

<center>TABLE J</center>

| | Two-Stage | One-Stage |
|---|---|---|
| Overall $C_3^=$ Conv. | 76.9 | 56.1 |
| Product Yields, wt% | | |
| Propylene | 23.1 | 43.9 |
| Isobutylene | 13.4 | 10.7 |
| Linear Butenes | 10.4 | 9.1 |
| Isoamylenes | 17.2 | 9.1 |
| Linear Pentenes | 6.6 | 3.6 |
| Total $iC_4^=$, $iC_5^=$ | 30.6 | 19.8 |
| Total $C_1$-$C_2$ | 0.2 | 0.4 |
| $C_3$-$C_5$ Paraffins | 2.0 | 3.1 |
| Total $C_6$ | 27.1 | 20.2 |

As seen in Table J, the isobutylene and isoamylene yield observed during this two-stage propylene conversion experiment was significantly higher than that obtained for the one-stage process (30.6 vs. 19.8 wt%).

**Claims**

1.  A process for oligomerizing an alkene-containing feedstock by contacting the feedstock with a catalyst comprising an inorganic, porous, non-layered crystalline phase material exhibiting, after calcination, an X-ray diffraction pattern with at least one peak at a d-spacing greater than about 18 Angstrom Units characterized in that said material has after calcination, a benzene adsorption capacity of greater than 15 grams of benzene per 100 grams of said material at 6.7 kPa (50 torr) and 25°C, has a hexagonal arrangement of uniformly-sized pores having diameters of at least about 13 Angstrom Units and, after calcination, exhibits a hexagonal electron diffraction pattern that can be indexed with a $d_{100}$ value greater than about 18 Angstrom Units.

2.  The process of claim 1 wherein said crystalline phase has a composition expressed as follows:

$$M_{n/q}(W_a X_b Y_c Z_d O_h)$$

    wherein M is one or more ions; n is the charge of the composition excluding M; q is the weighted molar average valence of M; n/q is the number of moles or mole fraction of M; W is one or more divalent elements; X is one or more trivalent elements; Y is one or more tetravalent elements; Z is one or more pentavalent elements; a, b, c, and d are mole fractions of W, X, Y, and Z, respectively; h is a number of from 1 to 2.5; and (a+b+c+d) = 1 .

3.  The process of claim 2 wherein the sum (a+b+c) is greater than d, and h = 2.

4.  The process of claim 2 wherein W comprises a divalent first row transition metal or magnesium; X comprises aluminum, boron, gallium or iron; Y comprises silicon or germanium; and Z comprises phosphorus.

5.  The process of claim 2 wherein a and d are 0 and h = 2; and wherein X comprises aluminum and Y comprises silicon.

6.  The process of claim 1 wherein the oligomerization is conducted at a temperature of 40 to 250°C; pressure of 100 - 13,000 kPa range; and a weight hourly space velocity, based on active catalyst, of 0.1-5.

7.  The process of claim 1 wherein the feedstock comprises $C_3$-$C_5$ alkenes and the oligomerization product contains $C_6^+$ hydrocarbons, the process further comprising the step of contacting the $C_6^+$ hydrocarbons with an intermediate pore zeolite catalyst to produce $C_4$-$C_5$ tertiary alkenes.

8.  The process of claim 7 wherein the intermediate pore zeolite catalyst comprises MCM-22 zeolite

**Patentansprüche**

1.  Verfahren zur Oligomerisierung eines Alken enthaltenden Beschickungsmaterials durch den Kontakt dieses Beschickungsmaterials mit einem Katalysator, der ein anorganisches, poröses, nicht geschichtetes Material mit kristalliner Phase umfaßt, das nach dem Kalzinieren ein Röntgenbeugungsdiagramm mit mindestens einem Peak bei einem d-Abstand von mehr als etwa 18 Angström-Einheiten aufweist, dadurch gekennzeichnet, daß dieses Material nach dem Kalzinieren eine Adsorptionskapazität für Benzol bei 6,7 kPa (50 Torr) und 25°C von mehr als 15 g Benzol pro 100 g des Materials aufweist, eine hexagonale Anordnung von Poren mit einheitlicher Größe besitzt, die Durchmesser von mindestens etwa 13 Angström-Einheiten haben, und nach dem Kalzinieren ein hexagonales Elektronenbeugungsdiagramm zeigt, das mit einem $d_{100}$-Wert von mehr als etwa 18 Angström-Einheiten indexiert werden kann.

2.  Verfahren nach Anspruch 1, wobei die kristalline Phase eine wie folgt ausgedrückte Zusammensetzung hat:

$$M_{n/q}(W_a X_b X_c Z_d O_h)$$

    worin M ein oder mehrere Ionen darstellt, n die Ladung der Zusammensetzung, M ausgenommen, ist; q der gewichtete Mittelwert der molaren Valenz von M ist; n/q die Anzahl der Mole oder der Molenbruch von M ist; W ein oder mehrere zweiwertige Elemente darstellt; X ein oder mehrere dreiwertige Elemente darstellt; Y ein oder mehrere vierwertige Elemente darstellt; Z ein oder mehrere fünfwertige Elemente darstellt; a, b, c, und d die Molenbrüche von W, X, Y bzw. Z sind; h eine Zahl von 1 bis 2,5 ist und $(a+b+c+d) = 1$ ist.

3. Verfahren nach Anspruch 2, wobei die Summe $(a+b+c)$ größer als d ist, und $h = 2$ ist.

4. Verfahren nach Anspruch 2, wobei W ein zweiwertiges Übergangsmetall der ersten Reihe oder Magnesium umfaßt, X Aluminium, Bor, Gallium oder Eisen umfaßt, Y Silicium oder Germanium umfaßt und Z Phosphor umfaßt.

5. Verfahren nach Anspruch 2, wobei a und d 0 sind und $h = 2$ ist, und wobei X Aluminium und Y Silicium umfassen.

6. Verfahren nach Anspruch 1, wobei die Oligomerisierung bei einer Temperatur von 40 bis 250°C, einem Druck im Bereich von 100 bis 13.000 kPa und einer stündlichen Gewichts-Raum-Geschwindigkeit, auf den aktiven Katalysator bezogen, von 0,1 bis 5 erfolgt.

7. Verfahren nach Anspruch 1, wobei das Beschickungsmaterial $C_3$-$C_5$-Alkene umfaßt und das Oligomerisierungsprodukt $C_{6+}$-Kohlenwasserstoffe enthält, wobei das Verfahren außerdem den Schritt des Kontaktes der $C_{6+}$-Kohlenwasserstoffe mit einem Zeolithkatalysator mit mittleren Poren umfaßt, wodurch tertiäre $C_4$-$C_5$-Alkene hergestellt werden.

8. Verfahren nach Anspruch 7, wobei der Zeolithkatalysator mit mittleren Poren den Zeolith MCM-22 umfaßt.

**Revendications**

1. Un procédé d'oligomérisation d'une charge contenant de l'alkène par mise de la charge au contact d'un catalyseur comprenant un matériau inorganique, en phase cristalline, non stratifiée, poreuse, présentant après calcination un spectre de diffraction aux rayons-X comportant au moins un pic à une distance-d supérieure à environ 18 Angstroems, caractérisé en ce que ledit matériau présente, après calcination, une capacité d'adsorption du benzène supérieure à 15 grammes de benzène par 100 grammes dudit matériau à 6,7 kPa (50 Torr) et 25°C, présente une disposition hexagonale de pores de dimension uniforme dont les diamètres sont au moins égaux à 13 Angstroems et, après calcination, présente un spectre de diffraction électronique hexagonale pouvant être indexé à une valeur du $d_{100}$ supérieur à environ 18 Angstroems.

2. Le procédé selon la revendication 1, dans lequel ladite phase cristalline présente une composition exprimée comme suit :

$$M_{n/q}(W_a\,X_b\,Y_c\,Z_d\,O_h)$$

dans laquelle :

| | |
|---|---|
| M | représente un ou plusieurs ions; |
| n | est la charge de la composition, sans tenir compte de M ; |
| q | représente la valence moyenne molaire pondérée de M; |
| n/q | représente le nombre de moles ou la fraction moléculaire de M ; |
| W | représente un ou plusieurs éléments divalents ; |
| X | représente un ou plusieurs éléments trivalents ; |
| Y | représente un ou plusieurs éléments tétravalents; |
| Z | représente un ou plusieurs éléments pentavalents; |
| a, b, c et d | sont les fractions molaires de W, X, Y et Z respectivement; |
| h | est un nombre compris entre 1 et 2,5 ; et |
| | $(a+b+c+d)=1$ . |

3. Le procédé selon la revendication 2, dans lequel la somme $(a+b+c)$ est supérieure à d, et h est égal à 2.

4. Le procédé selon la revendication 2, dans lequel W comprend un métal de transition divalent de la première ligne ou du magnésium, X comprend l'aluminium, le bore, le gallium ou le fer, Y comprend le silicium ou le germanium et Z comprend le phosphore.

**5.** Le procédé selon la revendication 2, dans lequel a et d sont 0 et h est égal à 2 ; et dans lequel X comprend l'aluminium et Y comprend le silicium.

**6.** Le procédé selon la revendication 1, dans lequel l'oligomérisation est conduite à une température de 40 à 250°C, une pression de 100 à 13 000 kPa et une vitesse spatiale horaire pondérale de 0,1 à 5 exprimée par rapport au catalyseur actif.

**7.** Le procédé selon la revendication 1, dans lequel la charge comprend des alkènes en $C_3$ à $C_5$ et le produit d'oligomérisation comprend des hydrocarbures en $C_6^+$, le produit comprenant en outre l'étape de mise en contact des hydrocarbures en $C_6^+$ avec un catalyseur à base de zéolite de pore intermédiaire pour produire des alkanes tertiaires en $C_4$ à $C_5$.

**8.** Le procédé selon la revendication 7, dans lequel le catalyseur zéolitique de pore intermédiaire comprend une zéolite MCM-22.